# NEW EUROPEAN PATENT SPECIFICATION

(11) **EP 2 046 268 B2**
(45) Date of publication and mention of the opposition decision: **13.07.2016**
(45) Mention of the grant of the patent: 27.02.2013
(21) Application number: 07788033.4
(22) Date of filing: 30.07.2007
(51) Int. Cl.: A61Q 11/00, A61K 8/11

(54) **ORAL CARE COMPOSITION**
MUNDPFLEGEZUSAMMENSETZUNG
COMPOSITIONS DE SOIN BUCCO-DENTAIRE

(30) Priority: 03.08.2006 EP 06118389
(43) Date of publication of application: 15.04.2009
(73) Proprietor: Unilever PLC, London EC4Y 0DY (GB); Unilever N.V., 3013 AL Rotterdam (NL)
(72) Inventor: GROVES, Brian Joseph, Bebington Wirral, Merseyside CH63 3JW (GB)
(74) Representative: Potter Clarkson LLP
(86) International application number: PCT/EP2007/057829
(87) International publication number: WO 2008/015186

(56) References cited:
- WO-A-2005/123023
- FR-A1- 2 124 311
- GB-A- 1 381 444
- US-A- 3 839 064
- US-A- 3 914 405
- US-A- 3 929 988
- US-A- 4 071 614
- US-A- 4 196 189
- US-A- 4 202 878
- US-A- 5 700 449
- US-A- 6 060 084
- US-A1- 2002 048 553
- US-B1- 6 238 690

## Description

The present invention relates to an oral care composition comprising a pigment and also to a method for manufacturing capsules comprising a pigment.

WO 2005/123023 A discloses toothpaste compositions comprising a dye encapsulated in wax, so that a colour change takes place when the capsule wall breaks during brushing.

In a first aspect the present invention there is provided an oral care composition comprising from 0.01 to 50% by weight of the composition of a capsule, the capsule comprising a core and a coating for the core, the coating comprising a wax having a melting point of at least 70 degrees C, an oil and an opacifier and the core comprising an oil, a pigment and a shear-thinning structurant, wherein the weight ratio between the wax and the oil in the coating is from 20:80 to 80:20, preferably from 30:70 to 40:60. Such a composition provides a useful indication to the user that toothbrushing has occurred for a predetermined time, for example, the professionally recommended two minutes.

In a second aspect of the present invention, there is provided a method of manufacturing capsules comprising pigment, said method comprising dispersing a pigment in oil and adding a shear-thinning structurant to gel the dispersion; heating an oil, a wax of melting point of at least 70 degrees C, and an opacifier to form a free flowing dispersion of the opacifier in molten wax/oil; and encapsulating the gelled dispersion of pigment in oil within the dispersion of opacifier in molten wax/oil, wherein the molten wax/oil ratio is from 20:80 to 80:20, preferably from 30:70 to 40:60.

The nature of the capsules used in the present invention enables good containment of pigment within the capsule until shear caused during toothbrushing leads to the breakdown of the capsules and the release of the pigment. For the capsules to function in the correct manner, showing colour only after the desired time, both the nature of the coating and the nature of the core of the capsules are important. Thus, it is important that the coating comprises a wax as described herein, an oil, and an opacifier and it is important that the core comprises an oil, a pigment, and a shear-thinning structurant.

The capsules are present in an amount ranging from 0.01 to 50% by weight of the composition. Preferably, they comprise from 0.5 to 10% by weight of the composition. Such an amount provides the optimum sensory during use and also the optimum pigment release profile when the capsules are broken during toothbrushing.

Typically, the coating of the capsules comprises from 20 to 60% of the total weight of the capsules, preferably from 30 to 50%, and more preferably around 40%.

The wax present in the coating is preferably one with a melting point of greater than 70 degrees C, more preferably greater than 80 degrees C. Such waxes include carnauba wax, Chinese insect wax, candililla wax, castor wax, Japan wax, shellac, ouricouri and synthetic waxes such as high molecular weight paraffin wax and microcrystalline wax.

The capsule coating preferably comprises from 20 to 60% by weight of wax with a melting point of greater than 70 degrees C and more preferably from 30 to 50%.

The oil, both in the coating and in the core, is preferably obtained from an animal, vegetable or mineral source. Preferred sources include vegetable sources such as fruits and plant seeds. Preferred oils include olive oil, borage oil, primrose oil, groundnut oil, canola oil, safflower oil, rice bran oil, peanut oil and other plant seed oils including sunflower (seed) oil. The most preferred sunflower oil is the so-called high-oleate sunflower oil which presents a higher resistance to rancidity.

The oil in the coating may be different to the oil in the core.

The capsule coating preferably comprises from 35 to 75% by weight of oil and more preferably from 50 to 65%.

The ratio between the wax and the oil in the coating is from 20:80 to 80:20, preferably from 30:70 to 40:60. These ratios provide the capsule with the best resistance to fracture during processing and also the best pigment release profile.

Preferably the opacifier is present at an amount to mask any colour in the core, preferably this is from 1 to 10% by weight of the coating, more preferably from 3 to 7% by weight of the coating. Preferably the opacifier is titanium dioxide.

Typically, the core comprises from 40 to 80% by weight of the capsule, preferably from 50 to 70% and more preferably around 60% by weight of the capsule.

The oil is present at from 50 to 95% by weight of the core, preferably from 60 to 90 and more preferably from 70 to 80% by weight of the core.

The core also comprises a pigment. The preferred pigments are blue, red, green, yellow, purple or orange. The most preferred are blue. Especially preferred are those selected from CI 14720, CI 15985, CI 16035, CI 16255, CI 19140, CI 42051, CI 42053, CI 42090, CI 47005, CI 73360, CI 74160, CI 74260, CI 75470, CI 75810, CI 77007, CI 77491 and CI 77492. These pigments present the best sensory profile during toothbrushing.

The pigment is present at from 1 to 50% by weight of the core, preferably from 10 to 30% by weight of the core.

The shear-thinning structurant present in the core of the capsules is important in achieving the desired delayed release of pigment. The presence of the shear-thinning structurant also assists the manufacture of the capsules used in the present invention.

The shear-thinning structurant is present in the core at from 1 to 40% by weight of the core, preferably from 2 to 10 and more preferably from 4 to 6% by weight of the core.

A preferred shear-thinning structurant is fumed silica. The capsules preferably have a weight average particle size of from 350 to 800 µm, more preferably from 400 to 600 µm. These present the best sensory to the user during toothbrushing.

The capsules according to the invention provide the best pigment loading without compromising capsule integrity since the highly viscous interior core is unlikely to leak out of a capsule when the coating is breached. The pigment will only leak out when the capsule is completely ruptured, i.e. during brushing.

The oral composition preferably comprises not more than 0.3% by weight pigment based on its powder weight, preferably not more than 0.2% by weight.

The oral composition according to the invention may comprise further ingredients which are common in the art, such as:
antimicrobial agents, e.g. Triclosan, chlorhexidine, copper, zinc, and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including capsuled particulate abrasive materials, usually in amounts between 3 and 60% by weight of the oral care composition.
humectants such as glycerol, sorbitol, propyleneglycol, xylitol, lactitol etc.;
binders and thickeners such as sodium carboxymethylcellulose, xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included. Examples of such polymers are copolymers of polyvinylmethylether with maleic anhydride and other similar delivery enhancing polymers, e.g. those described in DE-A-3,942,643 (Colgate);
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

The oral compositions may be in any form common in the art, e.g. toothpaste, gel, mousse, aerosol, gum, lozenge, powder, cream, etc. and may also be formulated into systems for use in dual-compartment type dispensers.

### EXAMPLES

Table 1 indicates the formulations of capsules as used in accordance with the present invention. Amounts of components are percentages by weight and the ratios of core:coating indicated are also by weight.

**Table 1**

| | **Example** | | | |
|---|---|---|---|---|
| | **1** | **2** | **3** | **4** |
| Core: | | | | |
| Olive oil | -- | -- | 72.5 | -- |
| Sunflower oil | 75 | 70 | -- | 80 |
| Pigment | 20 | 24 | 23.5 | 14 |
| Fumed silica | 5 | 6 | 4 | 6 |
| Coating: | | | | |
| Castor wax | -- | -- | 40 | -- |
| Carnauba wax | 38.4 | 32 | -- | 45 |
| Sunflower oil | 57.6 | 65 | 53 | 50 |
| Titanium dioxide | 4 | 3 | 7 | 5 |
| Core:coating | 50:50 | 40:60 | 70:30 | 60:40 |

The following process was used to make the capsules of Example 1.

Pigment (10g) was dispersed in sunflower oil (28.8g) using an overhead stirrer. Hydrophobic fumed silica (2.5g) was then added to the dispersion in order to gel it.

In a separate vessel, carnauba wax (19.2g) was heated to 85-90°C in order to melt it and sunflower oil (28.8g) and titanium dioxide (2g) were then mixed in using an overhead stirrer.

The gelled dispersion of pigment and sunflower oil was stirred into water (4L) held at 85-90°C. To this mixture was added the hot carnauba wax, sunflower oil, titanium dioxide mixture, with stirring. When this addition was complete, the aqueous suspension of encapsulates was rapidly cooled to below the melting point of the carnauba wax, the encapsulates were extracted, and dried.

In an alternative process of manufacture, applicable to all co-extrusion encapsulation is used. In this process, there are two hot feeds (jacketed lines or electrical heat traced) that feed, via controllable pumps, to a co-extrusion nozzle consisting of an internal tube carrying core material and an external tube carrying the coating material. The nozzle generally has a heating means in order to prevent solidification of the molten wax in the coating.

To control the particle size of the capsules, a means for breaking up the dual stream of hot materials exiting the co-extrusion nozzle is employed. Typical means are vibratory or use pressurised, heated air.

To solidify the encapsulates, a cooling means is employed. A typical means is a cooling tunnel, although a cooled water "collection bath" may alternatively or additionally be employed.

## Claims

1. An oral care composition comprising from 0.01 to 50% by weight of the composition of a capsule, the capsule comprising a core and a coating for the core, the coating comprising a wax having a melting point of at least 70 degrees C, an oil and an opacifier and the core comprising an oil, a pigment and a shear-thinning structurant, wherein the weight ratio between the wax and the oil in the coating is from 20:80 to 80:20, preferably from 30:70 to 40:60.

2. An oral care composition according to claim 1 wherein the wax has a melting point of at least 80 degrees C.

3. An oral care composition according to claim 1 or 2 wherein the oil is sunflower oil.

4. An oral care composition according to any preceding claim wherein the oil is high-oleate sunflower oil.

5. An oral care composition according to any preceding claim wherein the wax is carnauba wax.

6. An oral care composition according to any preceding claim wherein the opacifier is titanium dioxide.

7. An oral care composition according to any preceding claim wherein the shear thinning structurant is fumed silica.

8. An oral care composition according to any preceding claim wherein the pigment is selected from - CI 14720, CI 15985, CI 16035, CI 16255, CI 19140, CI 42051, CI 42053, CI 42090, CI 47005, CI 73360, CI 74160, CI 74260, CI 75470, CI 75810, CI 77007, CI 77491 and CI 77492.

9. An oral care composition according to any preceding claim wherein the weight average particle size of the capsules is from 350 to 800 µm, preferably from 400 to 600 µm.

10. An oral care composition according to any preceding claim wherein the total amount of pigment is not greater than 0.3% by weight, preferably not more than 0.2% by weight.

11. An oral care composition according to any preceding claim wherein the composition comprises a first phase and a second phase and wherein the capsule is present in only the first phase.

12. A method of manufacturing capsules comprising pigment, said method comprising dispersing a pigment in oil and adding a shear-thinning structurant to gel the dispersion; heating an oil, a wax of melting point of at least 70 degrees C, and an opacifier to form a free flowing dispersion of the opacifier in molten wax/oil; and encapsulating the gelled dispersion of pigment in oil within the dispersion of opacifier in molten wax/oil, wherein the molten wax/oil weight ratio is from 20:80 to 80:20, preferably from 30:70 to 40:60.

## Patentansprüche

1. Mundpflegezusammensetzung, die 0,01 bis 50 Gew.-% einer Kapsel, bezogen auf die Zusammensetzung, umfasst, wobei die Kapsel einen Kern und eine Beschichtung für den Kern umfasst, die Beschichtung ein Wachs mit einem Schmelzpunkt von wenigstens 70 °C, ein Öl und ein Trübungsmittel umfasst und der Kern ein Öl, ein Pigment und ein scherverdünnendes Strukturierungsmittel umfasst, wobei das Gewichtsverhältnis zwischen dem Wachs und dem Öl in der Beschichtung 20 : 80 bis 80 : 20, vorzugsweise 30 : 70 bis 40 :60, beträgt.

2. Mundpflegezusammensetzung gemäß Anspruch 1, wobei das Wachs einen Schmelzpunkt von wenigstens 80 °C hat.

3. Mundpflegezusammensetzung gemäß Anspruch 1 oder 2, wobei das Öl Sonnenblumenöl ist.

4. Mundpflegezusammensetzung gemäß einem vorangehenden Anspruch, wobei das Öl Sonnenblumenöl mit hohem Oleatgehalt ist.

5. Mundpflegezusammensetzung gemäß einem vorangehenden Anspruch, wobei das Wachs Carnaubawachs ist.

6. Mundpflegezusammensetzung gemäß einem vorangehenden Anspruch, wobei das Trübungsmittel Titandioxid ist.

7. Mundpflegezusammensetzung gemäß einem vorangehenden Anspruch, wobei das scherverdünnende Strukturierungsmittel hochdisperses Siliciumdioxid ist.

8. Mundpflegezusammensetzung gemäß einem vorangehenden Anspruch, wobei das Pigment ausgewählt ist aus CI 14720, CI 15985, CI 16035, CI 16255, CI 19140, CI 42051, CI 42053, CI 42090, CI 47005, CI 73360, CI 74160, CI 74260, CI 75470, CI 75810, CI 77007, CI 77491 und CI 77492.

9. Mundpflegezusammensetzung gemäß einem vorangehenden Anspruch, wobei die gewichtsmittlere Partikelgröße der Kapseln 350 bis 800 µm, vorzugsweise 400 bis 600 µm, beträgt.

10. Mundpflegezusammensetzung gemäß einem vorangehenden Anspruch, wobei die Gesamtmenge an Pigment nicht größer als 0,3 Gew.-%, vorzugsweise nicht größer als 0,2 Gew.-%, ist.

11. Mundpflegezusammensetzung gemäß einem vorangehenden Anspruch, wobei die Zusammensetzung eine erste Phase und eine zweite Phase umfasst und wobei die Kapsel nur in der ersten Phase vorhanden ist.

12. Verfahren zur Herstellung von Kapseln, die Pigment umfassen, wobei das Verfahren Dispergieren eines Pigments in Öl und Zusetzen eines scherverdünnenden Strukturierungsmittels, um die Dispersion zu gelieren, Erwärmen eines Öls, eines Wachses mit einem Schmelzpunkt von wenigstens 70 °C und eines Trübungsmittels unter Bildung einer frei fließenden Dispersion des Trübungsmittels in geschmolzenem Wachs/Öl und Einkapseln der gelierten Pigmentdispersion in Öl innerhalb der Dispersion von Trübungsmittel in geschmolzenem Wachs/Öl umfasst, wobei das Gewichtsverhältnis von geschmolzenem Wachs/Öl 20:80 bis 80:20, vorzugsweise 30:70 bis 40:60, beträgt.

## Revendications

1. Composition de soin buccal comprenant de 0,01 à 50 % en poids de la composition d'une capsule, la capsule comprenant un noyau et un enrobage pour le noyau, l'enrobage comprenant une cire ayant un point de fusion d'au moins 70 °C, une huile et un opacifiant et le noyau comprenant une huile, un pigment et un structurant de fluidification par cisaillement, dans laquelle le rapport en poids entre la cire et l'huile dans le revêtement est de 20:80 à 80:20, de préférence de 30:70 à 40:60.

2. Composition de soin buccal selon la revendication 1, dans laquelle la cire a un point de fusion d'au moins 80 °C.

3. Composition de soin buccal selon la revendication 1 ou 2, dans laquelle l'huile est une huile de tournesol.

4. Composition de soin buccal selon l'une quelconque des revendications précédentes, dans laquelle l'huile est une huile de tournesol à forte teneur en oléate.

5. Composition de soin buccal selon l'une quelconque des revendications précédentes, dans laquelle la cire est la cire de carnauba.

6. Composition de soin buccal selon l'une quelconque des revendications précédentes, dans laquelle l'opacifiant est le dioxyde de titane.

7. Composition de soin buccal selon l'une quelconque des revendications précédentes, dans laquelle le structurant de fluidification par cisaillement est la silice pyrogénée.

8. Composition de soin buccal selon l'une quelconque des revendications précédentes, dans laquelle le pigment est choisi parmi CI 14720, CI 15985, CI 16035, CI 16255, CI 19140, CI 42051, CI 42053, CI 42090, CI 47005, CI 73360, CI 74160, CI 74260, CI 75470, CI 75810, CI 77007, CI 77491 et CI 77492.

9. Composition de soin buccal selon l'une quelconque des revendications précédentes, dans laquelle la taille des particules moyenne en poids des capsules est de 350 à 800 µm, de préférence de 400 à 600 µm.

10. Composition de soin buccal selon l'une quelconque des revendications précédentes, dans laquelle la quantité totale de pigment n'est pas supérieure à 0,3 % en poids, de préférence n'est pas supérieure à 0,2 % en poids.

11. Composition de soin buccal selon l'une quelconque des revendications précédentes, dans laquelle la composition comprend une première phase et une seconde phase et dans laquelle la capsule est présente uniquement dans la première phase.

12. Procédé de fabrication de capsules comprenant un pigment, ledit procédé comprenant la dispersion d'un pigment dans une huile et l'ajout d'un structurant de fluidification par cisaillement pour gélifier la dispersion ; le chauffage d'une huile, d'une cire de point de fusion d'au moins 70 °C et d'un opacifiant pour former une dispersion fluide de l'opacifiant dans un mélange huile/cire fondue ; et l'encapsulation de la dispersion gélifiée de pigment dans une 'huile à l'intérieur de la dispersion d'opacifiant dans un mélange huile/cire fondue, dans laquelle le rapport en poids de cire fondue/huile est de 20:80 à 80:20, de préférence de 30:70 à 40:60.
